(19) Europäisches Patentamt
European Patent Office
Office européen des brevets

(11) **EP 2 700 354 B1**

(12) **EUROPÄISCHE PATENTSCHRIFT**

(45) Veröffentlichungstag und Bekanntmachung des Hinweises auf die Patenterteilung:
**25.07.2018   Patentblatt 2018/30**

(21) Anmeldenummer: **13173490.7**

(22) Anmeldetag: **25.06.2013**

(51) Int Cl.:
***A61B 5/03*** *(2006.01)*

(54) **Drainage-Steuersystem sowie Drainagesystem mit einem derartigen Drainage-Steuersystem**

Drainage control system and drainage system using such a drainage control system

Système de commande de drainage et système de drainage doté d'un tel système de commande de drainage

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.08.2012   DE 102012214763**

(43) Veröffentlichungstag der Anmeldung:
**26.02.2014   Patentblatt 2014/09**

(73) Patentinhaber: **RAUMEDIC AG**
**95213 Münchberg (DE)**

(72) Erfinder:
• **Göhler, Karlheinz**
  **08297 Zwönitz (DE)**
• **Kunze, Hans Gerd**
  **08297 Zwönitz (DE)**
• **Wierick, Philipp**
  **08297 Zwönitz (DE)**
• **Steudel, Wolf-Ingo**
  **66421 Homburg (DE)**
• **Kiefer, Michael**
  **66459 Kirkel (DE)**
• **Eymann, Regina**
  **66459 Kirkel (DE)**
• **Antes, Sebastian**
  **67655 Kaiserslautern (DE)**
• **Welsch, Melanie**
  **67146 Diedesheim (DE)**
• **Leonhardt, Steffen**
  **52076 Aachen-Oberforstbach (DE)**
• **Misgeld, Berno**
  **52074 Aachen (DE)**
• **Radermacher, Klaus**
  **52222 Aachen Stolberg (DE)**
• **Walter, Marian**
  **52070 Aachen (DE)**
• **Elixmann, Inga**
  **52070 Aachen (DE)**
• **Goffin, Christine**
  **52064 Aachen (DE)**
• **Hansinger, Jens**
  **70376 Stuttgart (DE)**

(74) Vertreter: **Rau, Schneck & Hübner**
**Patentanwälte Rechtsanwälte PartGmbB**
**Königstraße 2**
**90402 Nürnberg (DE)**

(56) Entgegenhaltungen:
CN-B- 101 879 058     US-A1- 2007 276 264
US-B1- 6 248 080

• Scalzo,Hamilton,Hu: "Real-Time Analysis of Intracranial Pressure Waveform Morphology" In: Dr Ken-Shiung Chen (Ed.): "Advanced Topics in Neurological Disorders", 16. März 2012 (2012-03-16), InTech, XP002718319, ISBN: 978-953-51-0303-5 Seiten 99-126, * das ganze Dokument *

EP 2 700 354 B1

**Beschreibung**

Die Erfindung betrifft ein Drainage-Steuersystem zur Steuersignalerzeugung für eine Drainageeinrichtung zum Einsatz in einer Drainageleitung zum Führen einer Flüssigkeit aus einer Hirnkammer eines Patienten. Ferner betrifft die Erfindung ein Drainagesystem mit einem derartigen Drainage-Steuersystem.

Ein Drainagesystem mit einem Ventil als Drainageeinrichtung zum Einsatz in einer Drainageleitung zum Führen einer Flüssigkeit aus einer Hirnkammer eines Patienten ist bekannt aus der EP 2 221 083 A und der US 6,248,080 B1. Weitere Drainageeinrichtungen zur kontrollierten Ableitung von Gehirnflüssigkeit sind bekannt aus der EP 0 982 048 A1 und der DE 196 43 782 C1.

Ein Verfahren zum Analysieren von physiologischen Drucksignalen ist aus der US 2007/0276264 A1 bekannt.

Es ist eine Aufgabe der vorliegenden Erfindung, ein Drainage-Steuersystem der eingangs genannten Art derart weiterzubilden, dass eine möglichst präzise Drainage der Hirnkammer jeweils nur dann erfolgt, wenn dies medizinisch tatsächlich erforderlich ist.

Diese Aufgabe ist erfindungsgemäß gelöst durch ein Drainage-Steuersystem mit den im Anspruch 1 angegebenen Merkmalen.

Das Drainage-Steuersystem nutzt eine vom Drucksensor zeitlich aufgelöst aufgenommene Druckdaten-Sequenz und bestimmt eine Dauer, eine Phase und Formcharakteristika einer Hirndruck-Welle, die korreliert mit dem Herzschlag entsteht und deren Form während einer Wellenperiode insbesondere hirndruckabhängig ist. Bei der Wellenform-Analyse erfolgt eine Zuordnung der gemessenen Hirndruck-Welle zu Wellenform-Kandidaten.

Hieraus wird über eine statistische Auswertung der Zuordnungshäufigkeiten auf den momentanen Druck bzw. auf die Compliance in der Hirnkammer unabhängig von möglichen absoluten Messdrifts des Drucksensors geschlossen. Es folgt eine sichere Reaktion auf Hirndruck-Zustände, die eine Drainage erfordern, durch entsprechendes, gesteuertes Öffnen der Drainageeinrichtung. Auch eine Öffnungsweite und eine Öffnungs-Zeitdauer der Drainageeinrichtung kann aus dem Ergebnis der Wellenform-Analyse abgeleitet werden. Im Wellenform-Kandidatenspeicher sind mindestens zwei Wellenform-Kandidaten abgespeichert. Es können drei, vier, fünf oder noch mehr Wellenform-Kandidaten im Wellenform-Kandidatenspeicher abgelegt sein. Das Drainage-Steuersystem kann als Implantat und insbesondere kompakt in Form eines Mikrochips ausgeführt sein.

Ein Drucksensor nach Anspruch 2 hat sich in der Praxis bewährt.

Eine Datenerfassungseinheit nach Anspruch 3 stellt einen guten Kompromiss zwischen Genauigkeit der Messdatenerfassung Hardware-Erfordernissen dar.

Eine Herzfrequenz-Analyse mittels des Herzfrequenz-Analysemoduls kann durch eine direkte Überwachung einer Patienten-Pulsfrequenz geschehen. Alternativ ist eine Ausführung des Herzfrequenz-Analysemoduls nach Anspruch 4 möglich, bei der die vom Drucksensor erfassten Druckdaten zur Ermittlung der Pulsfrequenz herangezogen werden. Eine separate direkte Überwachung der Pulsfrequenz entfällt dann. Bei einer weiteren Variante kann eine direkte Pulsfrequenz-Überwachung zur Überprüfung eines Herzfrequenz-Analysewertes herangezogen werden, der aus den vom Drucksensor erfassten Hirnkammer-Innendruckdaten gewonnen wurde.

Ein Medianfilter nach Anspruch 5 und ein Tiefpassfilter nach Anspruch 6 haben sich für die Herzfrequenz-Analyse als besonders geeignet herausgestellt.

Eine Zähleinheit nach Anspruch 7 ist eine einfach realisierbare Komponente des Wellenform-Analysemoduls. Die Wellenform-Analyse kann anhand der erfassten Anzahl der Druck-Maxima bzw. anhand der erfassten Anzahl der Druck-Minima der Hirndruck-Welle erfolgen. Alternativ oder zusätzlich kann ein relativer Druckunterschied zwischen aufeinanderfolgenden Druck-Maxima bzw. ein zeitlicher Abstand zwischen aufeinanderfolgenden Druck-Maxima zur Wellenform-Analyse herangezogen werden. Auch eine Amplitude, also der Druckunterschied zwischen einem Druck-Minimum und einem zeitlich benachbarten Druck-Maximum, kann alternativ oder zusätzlich zur Wellenform-Analyse herangezogen werden.

Eine Auswerteeinrichtung nach Anspruch 8 lässt sich hard- und softwaremäßig besonders einfach gestalten.

Ein Lagesensor nach Anspruch 9 ermöglicht eine Einbeziehung einer momentanen Patientenlage in eine Steuersignal-Generation für die Drainageeinrichtung. Beispielsweise kann bei einer erfassten Patientenlageänderung vom Liegen zum Stehen einer unerwünschten Überdrainage, insbesondere durch eine entsprechende Vorsteuerung, entgegengewirkt werden.

Die Vorteile eines Drainagesystems nach Anspruch 10 entsprechen denen, die vorstehend unter Bezugnahme auf das erfindungsgemäße Drainage-Steuersystem bereits erläutert wurden.

Ein Flusssensor nach Anspruch 11 kann zur Überwachung einer Drainagemenge eingesetzt werden. Auch hierüber kann eine unerwünschte Überdrainage vermieden werden.

Ein mit der Drainageeinrichtung und der Drainage-Steuereinrichtung in Signalverbindung stehender Drucksensor nach Anspruch 12 kann zur Bestimmung eines Zustandes des Patienten über die Erfassung einer zeitlichen Hirndruckveränderung nach definierter Entnahme einer bestimmten Liquormenge aus der Hirnkammer (Elastance) genutzt werden.

Ein Ventil nach Anspruch 13 und eine Pumpe nach Anspruch 14 sind bevorzugte Varianten für die Drainageeinrichtung.

Ausführungsbeispiele der Erfindung werden nachfolgend anhand der Zeichnung näher erläutert. In dieser zeigen:

Fig. 1 schematisch die Anordnung eines medizinischen Drainagesystems mit einer Drainageeinrichtung zum Einsatz in einer Leitung zum Abführen von Liquor aus einer Hirnkammer in einen Bauchraum eines Patienten;

Fig. 2 schematisch Hauptkomponenten des Drainagesystems mit einem Drainage-Steuersystem, einer Drainage-Steuereinrichtung und der Drainageeinrichtung; und

Fig. 3 ein zeitliches Druck-Messdiagramm, umfassend etwa eineinhalb Hirndruck-Wellenperioden; und

Fig. 4 ein Ablaufschema bei einer Wellenform-Analyse einer gemessenen Hirndruck-Welle durch Kandidatenzuordnung.

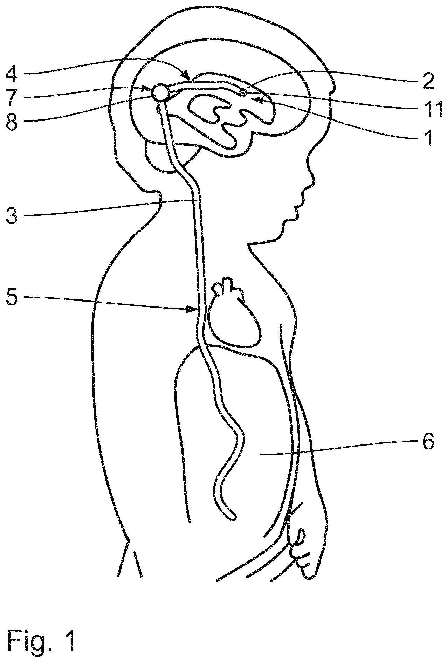

Fig. 1 zeigt schematisch einen Hydrozephalus-Patienten. Ein Ventrikelsystem 1 mit einem Liquorraum (Hirnkammer) 2 als Beispiel für eine Flüssigkeitskammer, nämlich für eine mit Flüssigkeit gefüllte Körperhöhlung, steht über eine Liquorleitung 3 mit einem proximalen Leitungsabschnitt 4 und einem distalen Leitungsabschnitt 5 mit einem Bauchraum 6 in Fluidverbindung. In der Liquorleitung 3 ist ein medizinisches Drainagesystem 7 mit einer Drainageeinrichtung in Form eines Ventils 8 angeordnet. Letzteres dient zum gezielten Abführen von Liquor als Beispiel für eine Flüssigkeit aus dem Liquorraum (Hirnkammer) 2. Das Drainagesystem 7 kann insgesamt als Implantat ausgeführt sein.

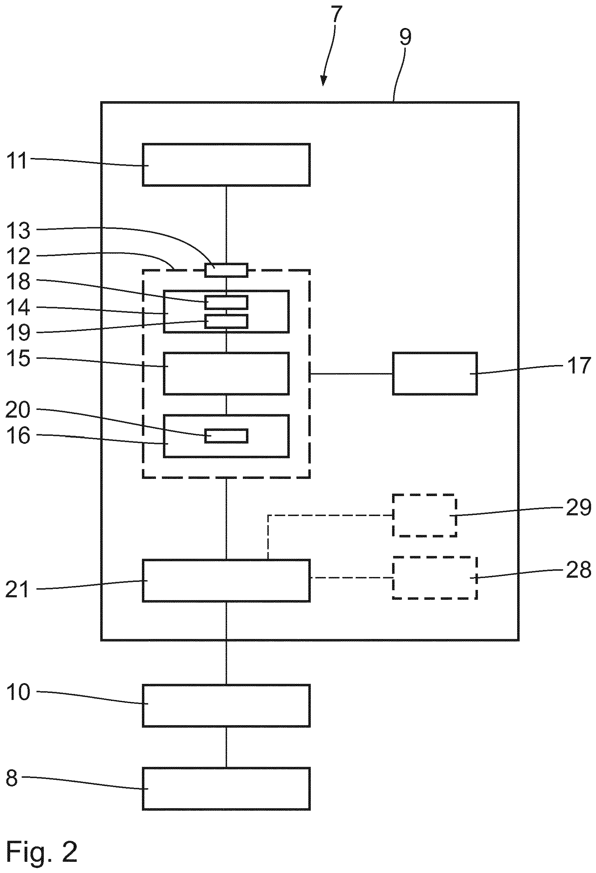

Fig. 2 zeigt Hauptkomponenten des Drainagesystems 7. Hierzu gehören ein Drainage-Steuersystem 9 zur Steuersignalerzeugung für das Ventil 8, eine Drainage-Steuereinrichtung 10, die mit dem Drainage-Steuersystem 9 in Signalverbindung steht, und das Ventil 8 selbst. Das Ventil 8 kann z. B. nach Art des Ventils ausgeführt sein, das beschrieben ist in der EP 2 221 083 A oder in der DE 196 43 782 C1.

Zum Drainage-Steuersystem 9 gehört ein Drucksensor 11 zur zeitaufgelösten Erfassung eines Innendrucks in der Hirnkammer 2. Der Drucksensor 11 steht in Signalverbindung mit einer Auswerteeinrichtung 12 des Drainage-Steuersystems 9. Der Drucksensor 11 ist in einer Spitze des proximalen Leitungsabschnitts 4 angeordnet, der als Hirnkatheter ausgeführt ist.

Teil der Auswerteeinrichtung 12 ist eine Datenerfassungseinheit 13 zur Erfassung einer Druckdaten-Sequenz vom Drucksensor 11 während eines Zeitraums im Bereich zwischen 3 Sekunden und 10 Sekunden. Eine Abtastfrequenz der Datenerfassungseinheit 13 kann mindestens 100 Hz betragen.

Bei der beschriebenen Ausführung misst die Datenerfassungseinheit 13 die Druckdaten-Sequenz während eines Zeitraums im Bereich von 5 Sekunden, beispielsweise während 5,12 Sekunden. Die Datenerfassungseinheit 13 ist als Datalogger ausgeführt.

Der Drucksensor 11 steht direkt mit der Datenerfassungseinheit 13 in Signalverbindung.

Zur Auswerteeinrichtung 12 gehören ein Herzfrequenz-Analysemodul 14 zur Bestimmung einer Patienten-Herzfrequenz aus der vom Drucksensor 11 übermittelten Druckdaten-Sequenz, ein Wellenphasen-Extraktionsmodul 15 zur Bestimmung eines Anfangszeitpunktes und eines Endzeipunktes einer zur Herzfrequenz synchronen Hirndruck-Welle und ein Wellenform-Analysemodul 16, welches eine Form der Hirndruck-Welle zwischen dem vom Wellenphasen-Extraktionsmodul 15 bestimmten Anfangs- und Endzeitpunkt einer bestimmten Wellenform aus einer Mehrzahl von Wellenform-Kandidaten zuordnet.

Die Wellenform-Kandidaten sind in einem Wellenform-Kandidatenspeicher 17 des Drainage-Steuersystems 9 abgelegt.

Das Herzfrequenz-Analysemodul 14 umfasst einen Medianfilter 18 und einen Tiefpassfilter 19. Bei dem Medianfilter 18 und dem Tiefpassfilter 19 handelt es sich jeweils um einen digitalen Filter.

Das Wellenform-Analysemodul 16 umfasst eine Zähleinheit 20 zum Zählen von Druck-Maxima innerhalb einer vom Drucksensor 11 erfassten und vom Wellenphasen-Extraktionsmodul 15 extrahierten Hirndruck-Welle.

Die Auswerteeinrichtung 12 ist als binär arbeitender Digitalrechner derart ausgeführt, dass Druck- und/oder Zeitgrenzwerte, die bei der Analyse insbesondere in den Modulen 14, 15, 16 genutzt werden, als Zweierpotenzen jeweiliger Grenz-Referenzwerte ausgewählt werden.

Zur Auswerteeinrichtung 12 gehört weiterhin ein Steuersignal-Generationsmodul 21, das mit der Auswerteeinrichtung 12 in Signalverbindung steht. Das Steuersignal-Generationsmodul 21 generiert ein Drainage-Steuersignal anhand eines Zuordnungsergebnisses des Wellenform-Analysemoduls 16 der Auswerteeinrichtung 12. Die Drainage-Steuereinrichtung 10 steht mit dem Steuersignal-Generationsmodul 21 in Signalverbindung.

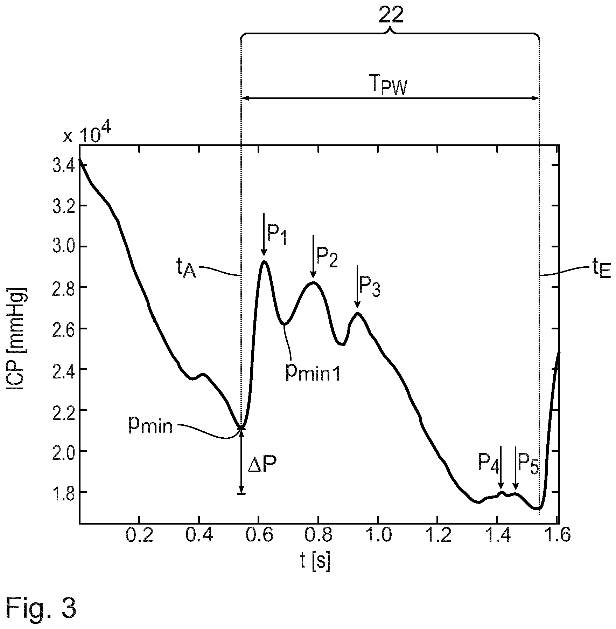

Kehrwert der vom Herzfrequenz-Analysemodul 14 bestimmten Patienten-Herzfrequenz ist eine Wellenperiode der Hirndruck-Welle 22 (vgl. Fig. 3), also ein Zeitraum zwischen dem Anfangszeitpunkt $t_A$ und dem Endzeitpunkt $t_E$, die mit dem Wellenphasen-Extraktionsmodul phasenrichtig bestimmt werden. Eine Bestimmung des Anfangszeitpunktes $t_A$ und des Endzeitpunktes $t_E$ erfolgt durch Vergleich aufeinanderfolgender Hirndruck-Wellen und durch Ermittlung eines Druck-Minimums innerhalb der Druckdaten-Sequenz der Hirndruck-Welle 22, das im Vergleich zu anderen Druck-Minima innerhalb der Hirndruck-Wellenperiode bestimmten Vorgaben genügt. Zu diesen Vorgaben kann beispielsweise ein Verwerfen aller Druck-Minima gehören, nach denen der Druck nicht über einen vorgegebenen Druck-Grenzwert ansteigt. Eine weitere Vorgabe kann ein Verwerfen aller Druck-Minima mit zu hohem absolutem Druckwert sein. Eine weitere Vorgabe kann ein Verwerfen von Druck-Minima sein, auf die ein Druck-Minimum mit zu geringem absolutem Druckwert folgt.

Eine Wellenform-Analyse im Wellenform-Analysemodul 16 erfolgt anhand einer Anzahl der erfassten Druck-Maxima innerhalb einer Druckdaten-Sequenz einer phasenrichtig bestimmten Hirndruck-Welle. Diese Wellenform-Analyse geschieht anhand eines relativen Druckunterschiedes zwischen aufeinanderfolgenden Druck-Maxima sowie anhand eines zeitlichen Abstandes zwischen aufeinanderfolgenden Druck-Maxima. Hierbei wird die Übereinstimmung der jeweils gemessenen Druckdaten-Sequenz einer Hirndruck-Welle mit den Charakteristika der im Kandidatenspeicher 17 abgelegten Wellenform-Kandidaten überprüft.

Nachfolgend wird eine Druckdaten-Auswertung näher erläutert.

Fig. 3 zeigt eine gemessene typische Druckdaten-Sequenz. Gezeigt ist ein Druckmesswert ICP (intracranial pressure, Hirndruck) während einer Messdauer von etwa 1,6 s. Teil der in der Fig. 2 dargestellten Druckdaten-Sequenz ist die Hirndruck-Welle 22 mit Wellenperiode $T_{PW}$. Die Hirndruck-Welle 22 hat fünf charakteristische Druck-Maxima $P_1$ bis $P_5$, die innerhalb der Hirndruck-Wellenperiode $T_{PW}$ aufeinanderfolgen. Dargestellt in der Fig. 3 ist auch der Anfangszeitpunkt $t_A$ und der Endzeitpunkt $t_E$ der Hirndruck-Welle 22, die periodisch synchron zur Herzfrequenz auftritt.

Der Anfangszeitpunkt $t_A$ wird durch Vergleich aufeinanderfolgender Hirndruck-Wellen 22 und durch Ermittlung eines Druck-Minimums $p_{min}$ bestimmt, das im Vergleich zu anderen Druck-Minima zwischen den Maxima $P_1$ bis $P_5$ folgenden Vorgaben genügt: Nach dem Minimum $p_{min}$ muss der Hirndruck über einen vorgegebenen Grenzwert steigen, beispielsweise über einen Wert von 2,5 mmHg. Dieser vorgegebene Grenzwert ist bezogen auf eine Wellenamplitude der jeweiligen Hirndruck-Welle 22, also auf den Unterschied zwischen einem maximalen Druckwert und einem minimalen Druckwert der Hirndruck-Welle 22. Diese Amplitude wird auch als P-Wellenamplitude bezeichnet. Der vorgegebene Grenzwert, über den der Hirndruck nach dem Minimum $p_{min}$ steigen muss, kann auch 25 % der P-Wellenamplitude betragen. Zudem muss auf das Druck-Minimum $p_{min}$ ein weiteres Druck-Minimum $p_{min1}$ folgen, dessen Druckwert deutlich höher ist als der Druckwert des Anfangszeit-Druck-Minimums $p_{min}$. Der Endzeitpunkt $t_E$ wird bestimmt, in dem alle Druck-Minima am zeitlichen Ende einer Hirndruck-Welle 22, welche einen bestimmten Druck-Schwellenwert überschreiten, verworfen werden, da diese dann einer nachfolgenden Hirndruck-Welle zuzuordnen sind.

Zwischen den Druckwerten $ICP(t_A)$ und $ICP(t_E)$ kann ein Druck-Offset $\Delta P$ aufgrund zusätzlicher physiologischer Randbedingungen vorliegen.

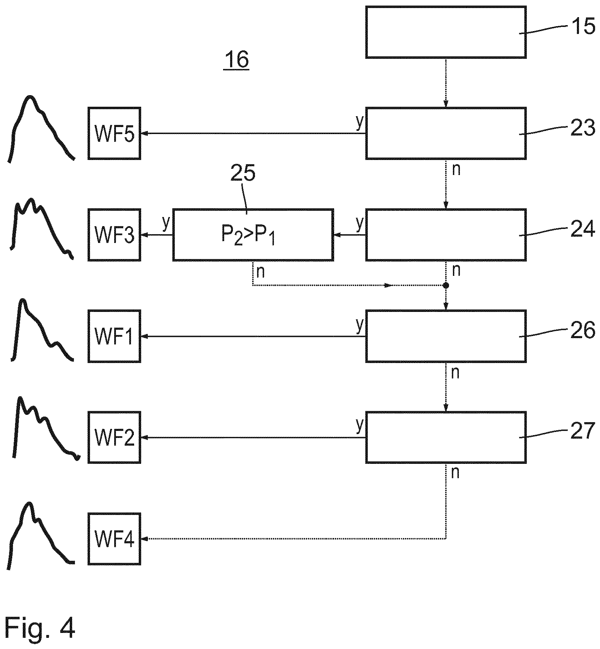

Die hinsichtlich ihrer Wellenperiode und ihrer Phase ausgewertete Hirndruck-Welle 22 wird dann hinsichtlich ihrer zeitlichen Form einer Analyse bestimmt. Dies wird nachfolgend anhand der Fig. 4 beschrieben. Die gemessene Hirndruck-Welle 22, beispielsweise diejenige nach Fig. 3, wird mit insgesamt fünf Kandidatenwerten (Typen) $WF_i$ verglichen, die in der Fig. 4 mit den Ziffern 1 bis 5 durchnummeriert sind.

Diese Hirndruck-Wellenformen Typen $WF_1$ bis $WF_5$ stellen typische Wellenformen dar, die bei Wellenform-Form von Typ 1 bis 5 zunehmendem Hirndruck und bei Wellenform-Form von Typ 1 bis 5 abnehmender Compliance C auftreten, wobei die Compliance definiert ist als

$$C(ICP) = I_{inf} \cdot \left( \frac{dICP(t_1)}{dt} + \frac{dICP(t_2)}{dt} \right)^{-1}.$$

Hierbei stellt $I_{inf}$ eine Infusionsrate dar, die bei einem Infusionstest beispielsweise mit einer Größe von 3 ml/min. der Hirnkammer 2 zugegeben wird. Die beiden Ableitungen des zeitlichen Hirndruck-Verlaufs ICP(t) in der Formel geben einen Anstieg des Hirndrucks während der Infusion beim Erreichen eines vorgegebenen Druckwertes $P_o$ und einen Abfall des Druckwertes nach Beendigung der Infusion beim gleichen Druckwert $P_0$ wieder.

Die fünf Wellenformen Typen 1 bis 5 nach Fig. 4 unterscheiden sich durch die Anzahl, die relative Höhe und die zeitliche Abfolge der Druck-Maxima. Bei der Wellenform-Analyse werden also die Anzahl der Druck-Maxima, der relative Druckunterschied zwischen aufeinanderfolgenden Druck-Maxima und ein zeitlicher Abstand zwischen aufeinanderfolgenden Druck-Maxima $P_i$ ausgewertet. Dies geschieht im Wellenform-Analysemodul 16. Dort wird die Druckdaten-Sequenz einer phasenrichtig extrahierten Hirndruck-Welle 22 zunächst hinsichtlich der Anzahl der Druck-Maxima analysiert. In einem ersten Abfrageschritt 23 wird zunächst überprüft, ob genau ein Druck-Maximum vorliegt. Falls ja ("y" in der Fig. 4), ist klar, dass die Wellenform Typ 5 vorliegt. Falls nein ("n" in der Fig. 4), wird nachfolgend in einem weiteren Abfrageschritt 24 abgefragt, ob die Anzahl der Druck-Maxima größer ist als 2. Falls ja, wird in einem weiteren Abfrageschritt 25 abgefragt, ob der Druckwert $P_2$ des zweiten Druck-Maximums innerhalb der gemessenen Hirndruck-Welle 22 größer ist als der Druckwert $P_1$ des ersten Druck-Maximums. Falls ja, entspricht die Wellenform der gemessenen Hirndruck-Welle 22 der Kandidatenform Typ 3. Falls nein oder für den Fall, dass der Abfrageschritt 24 das Ergebnis "nein" ergibt, wird in einem weiteren Abfrageschritt 26 abgefragt, ob der zeitliche Abstand zwischen dem ersten Druck-Maximum $P_1$ und einem nachfolgenden Druck-Maximum $P_2$ größer ist als ein vorgegebener Grenzwert, beispielsweise größer ist als ein Drittel der gesamten Wellenperiode der gemessenen Hirndruck-Welle 22. Ist dies der Fall, liegt die Kandidatenform Typ 1 vor. Falls nein, wird noch einmal das Ergebnis des Abfrageschritts 24 herangezogen. Hat die gemessene Hirndruck-Welle 22 mehr als zwei Druck-Maxima, liegt eine der Kandidatenform Typ 2 entsprechende Wellenform vor. Falls nicht, liegt eine der Kandidatenform Typ 4 entsprechende Wellenform vor.

Je nach der Häufigkeit der Kandidatenformen 1 bis 5, die sich bei der Zuordnung der jeweils gemessenen Hirndruck-Welle 22 ergibt, kann dann ein Steuersignal für die Ventil-Steuereinrichtung 10 vom Steuersignal-Generationsmodul 21 generiert werden. Das Ventil 8 kann dann geöffnet werden, wenn sich aufgrund der Zuordnung ergibt, dass ein entsprechendes Druckszenario vorliegt.

Bei einer Variante des Ventilsystems 7 kann das Ventil-Steuersystem 9 zusätzlich noch einen Lagesensor 28 zur Erfassung einer Lage des Patienten aufweisen, wie in der Fig. 2 gestrichelt angedeutet. Der Lagesensor 28 steht mit dem Steuersignal-Generationsmodul 21 in Signalverbindung. Der Lagesensor 28, der beispielsweise aufgebaut sein kann wie Lagesensoren, die aus Mobiltelefonen bekannt sind, erfasst eine Lage insbesondere eines Kopfes des Patienten und kann entscheiden, ob dieser steht, sich beugt oder liegt. Je nach der erfassten Lage des Patienten errechnet das Steuersignal-Generationsmodul 21 einen Korrekturwert zur Ansteuerung der Drainage-Steuereinrichtung 10.

Alternativ oder zusätzlich kann das Drainage-Steuersystem 9 einen Flusssensor 29 aufweisen, wie in der Fig. 2 ebenfalls gestrichelt angedeutet. Der Flusssensor 29 steht ebenfalls mit dem Steuersignal-Generationsmodul 21 in Signalverbindung. Der Flusssensor 29 misst einen Drainagefluss von Liquor durch das Ventil 8, also eine Liquormenge, die vom Ventil 8 pro Zeiteinheit bei der Drainage abgeleitet wird. Mit dem Flusssensor 29 kann eine maximale Drainagemenge pro Zeiteinheit begrenzt werden, was eine Überdrainage vermeidet. Hierzu gibt der Flusssensor 29 beim Erreichen eines vorgegebenen Drainagemengen-Grenzwerts ein entsprechendes Signal an das Steuersignal-Generationsmodul 21.

Anstelle eines Ventils 8 kann bei einer weiteren Variante des DrainageSystems 7 eine Pumpe zum Einsatz kommen, die, je nach Ansteuerung durch die Drainage-Steuereinrichtung 10, eine vorgegebene Menge Liquor über die Liquorleitung 3 in den Bauchraum 6 aktiv fördert. Als Steuerwert für die Pumpe wird eine vorgegebene Drainagemenge mit Hilfe des Steuersignal-Generationsmoduls 21 generiert.

Mit dem Drainagesystem 7 kann weiterhin ein Patientenzustand über eine weitere Kenngröße, die Elastance E, bestimmt werden. Die Elastance ist definiert

$$E(ICP) = dICP/dV.$$

Hierzu wird eine definierte Drainagemenge von Liquor in einem kurzen, definierten Zeitraum (sogenannter inverser Bolus) aus der Hirnkammer 2 entnommen und es wird eine resultierende Änderung des Hirndrucks ICP bestimmt.

**Patentansprüche**

1. Drainage-Steuersystem (9) zur Steuersignalerzeugung für eine Drainageeinrichtung (8) zum Einsatz in einer Drainageleitung (3) zum Führen einer Flüssigkeit aus einer Hirnkammer (2) eines Patienten

   - mit einem Drucksensor (11) zur zeitaufgelösten Erfassung eines Hirnkammer-Innendrucks,
   - mit einem Wellenform-Kandidatenspeicher (17),
   - mit einer Auswerteeinrichtung (12), die mit dem Drucksensor (11) und dem Wellenform-Kandidatenspeicher (17) in Signalverbindung steht,
   - wobei die Auswerteeinrichtung (12) aufweist:

      -- ein Herzfrequenz-Analysemodul (14) zur Bestimmung einer Patienten-Herzfrequenz,
      -- ein Wellenphasen-Extraktionsmodul (15) zur Bestimmung eines Anfangszeitpunktes ($t_A$) und eines Endzeitpunktes ($t_E$) einer zur Herzfrequenz synchronen Hirndruck-Welle (22),
      -- ein Wellenform-Analysemodul (16), welches eine Form der Hirndruck-Welle (22) zwischen dem bestimmten Anfangs- und Endzeitpunkt einer bestimmten Wellenform aus einer Mehrzahl von Wellenform-Kandidaten ($WF_1$-$WF_5$) zuordnet, die im Wellenform-Kandidatenspeicher (17) abgelegt sind,

   - mit einem Steuersignal-Generationsmodul (21), das mit der Auswerteeinrichtung (12) in Signalverbindung steht und ein Drainage-Steuersignal anhand eines Zuordnungsergebnisses des Wellenform-Analysemoduls (16) der Auswerteeinrichtung (12) generiert,
   - wobei eine Zuordnung derart erfolgt, dass über eine statistische Auswertung der Zuordnungshäufigkeiten ein Rückschluss auf den momentanen Druck und/oder auf eine Compliance in der Hirndruckkammer unabhängig von möglichen absoluten Messdrifts des Drucksensors erfolgt.

2. Steuersystem nach Anspruch 1, **dadurch gekennzeichnet, dass** der Drucksensor (11) in einer Katheterspitze eines Hirnkatheters (4) ausgeführt ist.

3. Steuersystem nach Anspruch 1 oder 2, **dadurch gekennzeichnet, dass** die Auswerteeinrichtung (12) eine Datenerfassungseinheit (13) zur Erfassung einer Druckdaten-Sequenz vom Drucksensor (11) während eines Zeitraums im Bereich zwischen 3 s und 10 s aufweist.

4. Steuersystem nach einem der Ansprüche 1 bis 3, **dadurch gekennzeichnet, dass** das Herzfrequenz-Analysemodul (14) zur Bestimmung der Patienten-Herzfrequenz aus einer vom Drucksensor (11) übermittelten Druckdaten-Sequenz ausgeführt ist.

5. Steuersystem nach einem der Ansprüche 1 bis 4, **dadurch gekennzeichnet, dass** das Herzfrequenz-Analysemodul (14) einen Medianfilter (18) aufweist.

6. Steuersystem nach einem der Ansprüche 1 bis 5, **dadurch gekennzeichnet, dass das** Herzfrequenz-Analysemodul (14) einen Tiefpassfilter (19) aufweist.

7. Steuersystem nach einem der Ansprüche 1 bis 6, **dadurch gekennzeichnet, dass** das Wellenform-Analysemodul (16) eine Zähleinheit (20) zum Zählen von Druck-Maxima ($P_1$, ...) innerhalb eines Hirndruck-Welle (22) aufweist.

8. Steuersystem nach einem der Ansprüche 1 bis 7, **dadurch gekennzeichnet, dass** die Auswerteeinrichtung (12) als binär arbeitender Digitalrechner derart ausgeführt ist, dass Druck- und/oder Zeitgrenzwerte, die bei der Analyse genutzt werden, als Zweierpotenzen jeweiliger Grenz-Referenzwerte ausgebildet werden.

9. Steuersystem nach einem der Ansprüche 1 bis 8, **gekennzeichnet durch** einen Lagesensor (28) zur Erfassung einer Lage des Patienten, der mit dem Steuersignal-Generationsmodul (21) in Signalverbindung steht.

10. Drainagesystem (7)

 - mit einem Drainage-Steuersystem (9) nach einem der Ansprüche 1 bis 9,
 - mit einer Drainage-Steuereinrichtung (10), die mit dem Steuersignal-Generationsmodul (12) in Signalverbindung steht,
 - mit einer Drainageeinrichtung (8) zum Einsatz in der Drainageleitung (3) zum Führen der Flüssigkeit aus der Hirnkammer (2) des Patienten,
 - wobei die Drainageeinrichtung (8) mit der Drainage-Steuereinrichtung (10) in Signalverbindung steht.

11. Drainagesystem nach Anspruch 10, **gekennzeichnet durch** einen Flusssensor zur Erfassung eines Flüssigkeits-Flusses durch die Drainageeinrichtung (8).

12. Drainagesystem nach Anspruch 10 oder 11, **dadurch gekennzeichnet, dass** der Drucksensor (11) mit der Drainageeinrichtung (8) und der Drainage-Steuereinrichtung (10) in Signalverbindung steht.

13. Drainagesystem nach einem der Ansprüche 10 bis 12, **dadurch gekennzeichnet, dass** die Drainageeinrichtung (8) als Ventil ausgeführt ist.

14. Drainagesystem nach einem der Ansprüche 10 bis 13, **dadurch gekennzeichnet, dass** die Drainageeinrichtung (8) als Pumpe ausgeführt ist.

**Claims**

1. Drainage control system (9) for control signal generation for a drainage unit (8) to be used in a drainage line (3) to remove a liquid from a patient's cerebral ventricle (2), comprising

 - a pressure sensor (11) for the time-resolved detection of a cerebral ventricle internal pressure,
 - a waveform candidate memory (17),
 - an evaluation unit (12), which is in signal communication with the pressure sensor (11) and the waveform candidate memory (17),
 - the evaluation unit (12) comprising

   -- a heart rate analysis module (14) to determine a patient's heart rate,
   -- a wave phase extraction module (15) to determine a starting time ($t_A$) and an end time ($t_E$) of an intracranial pressure wave (22) synchronous to the heart rate,
   -- a waveform analysis module (16), which allocates a form of the intracranial pressure wave (22) between the starting time and end time thus determined to a waveform determined from a plurality of waveform candidates ($WF_1$-$WF_5$), which are stored in the waveform candidate memory (17),

 - a control signal generation module (21), which is in signal communication with the evaluation unit (12) and generates a drainage control signal based on an allocation result of the waveform analysis module (16) of the evaluation unit (12),
 - wherein an allocation is carried out in such a way that by performing a statistical evaluation of the allocation frequencies, a conclusion is drawn as to the current pressure and/or a compliance in the cerebral ventricle irrespective of potential absolute measurement drifts of the pressure sensor.

2. Control system according to claim 1, **characterized**

**in that** the pressure sensor (11) is formed in a catheter tip of a cerebral catheter (4).

3. Control system according to claim 1 or 2, **characterized in that** the evaluation unit (12) has a data detection unit (13) to detect a pressure data sequence of the pressure sensor (11) during a time period in the range of between 3 s and 10 s.

4. Control system according to any one of claims 1 to 3, **characterized in that** the heart rate analysis module (14) is configured to determine the patient's heart rate from a pressure data sequence transmitted by the pressure sensor (11).

5. Control system according to any one of claims 1 to 4, **characterized in that** the heart rate analysis module (14) has a median filter (18).

6. Control system according to any one of claims 1 to 5, **characterized in that** the heart rate analysis module (14) has a low pass filter (19).

7. Control system according to any one of claims 1 to 6, **characterized in that** the waveform analysis module (16) has a counting unit (20) to count pressure maxima ($P_1$, ...) in a cerebral pressure wave (22).

8. Control system according to any one of claims 1 to 7, **characterized in that** the evaluation unit (12) is configured as a binary digital computer such that pressure and/or time limits used during analysis are configured as powers of two of respective reference limits.

9. Control system according to any one of claims 1 to 8, **characterized by** a position sensor (28) to detect a patient's position, the position sensor (28) being in signal communication with the control signal generation module (21).

10. Drainage system (7)

    - comprising a drainage control system (9) according to any one of claims 1 to 9,
    - comprising a drainage control unit (10), which is in signal communication with the control signal generation module (12),
    - comprising a drainage unit (8) to be used in the drainage line (3) to remove the liquid from the patient's cerebral ventricle (2),
    - wherein the drainage unit (8) is in signal communication with the drainage control unit (10).

11. Drainage system according to claim 10, **characterized by** a flow sensor to detect a liquid flow through the drainage unit (8).

12. Drainage system according to claim 10 or 11, **characterized in that** the pressure sensor (11) is in signal communication with the drainage unit (8) and the drainage control unit (10).

13. Drainage system according to any one of claims 10 to 12, **characterized in that** the drainage unit (8) is configured as a valve.

14. Drainage system according to any one of claims 10 to 13, **characterized in that** the drainage unit (8) is configured as a pump.

**Revendications**

1. Système de commande de drainage (9) prévu pour générer des signaux de commande d'un moyen de drainage (8) destiné à être utilisé dans un conduit de drainage (3) pour guider un liquide hors de la boîte crânienne (2) d'un patient, ledit système comprenant

    - un capteur de pression (11) servant à la détection à résolution temporelle de la pression intérieure de la boîte crânienne,
    - une mémoire de formes d'onde candidates (17),
    - un moyen d'évaluation (12) qui communique avec le capteur de pression (11) et la mémoire de formes d'onde candidates (17) par une liaison de signal,
    - le moyen d'évaluation (12) comprenant :

        -- un module d'analyse de fréquence cardiaque (14) servant à déterminer la fréquence cardiaque d'un patient,
        -- un module d'extraction de phase d'onde (15) servant à déterminer un instant initial ($t_A$) et un instant final ($t_E$) d'une onde de pression intracrânienne (22) synchrone de la fréquence cardiaque,
        -- un module d'analyse de forme d'onde (16) qui associe une forme d'onde de pression intracrânienne (22) entre l'instant initial et l'instant final déterminés à une forme d'onde déterminée parmi une pluralité de formes d'onde candidates ($WF_1$ à $WF_5$) stockées dans la mémoire de formes d'onde candidates (17),

        - un module de génération de signal de commande (21) qui communique avec le moyen d'évaluation (12) par une liaison de signal et qui génère un signal de commande de drainage sur la base d'un résultat d'association du module d'analyse de forme d'onde (16) du moyen d'évaluation (12),

- une association étant effectuée de telle sorte qu'une conclusion sur la pression actuelle et/ou sur une conformité dans la chambre de pression intracrânienne indépendamment de la dérive de mesure absolue possible du capteur de pression est effectuée grâce à une évaluation statistique des fréquences d'association.

2. Système de commande selon la revendication 1, **caractérisé en ce que** le capteur de pression (11) est réalisé dans une pointe de cathéter d'un cathéter crânien (4).

3. Système de commande selon la revendication 1 ou 2, **caractérisé en ce que** le moyen d'évaluation (12) comprend une unité d'acquisition de données (13) servant à acquérir une séquence de données de pression à l'aide du capteur de pression (11) pendant un intervalle de temps compris entre 3 s et 10 s.

4. Système de commande selon l'une des revendications 1 à 3, **caractérisé en ce que** le module d'analyse de fréquence cardiaque (14) est conçu pour déterminer la fréquence cardiaque du patient à partir d'une séquence de données de pression transmise par le capteur de pression (11).

5. Système de commande selon l'une des revendications 1 à 4, **caractérisé en ce que** le module d'analyse de fréquence cardiaque (14) comporte un filtre passe-bande (18).

6. Système de commande selon l'une des revendications 1 à 5, **caractérisé en ce que** le module d'analyse de fréquence cardiaque (14) comporte un filtre passe-bas (19).

7. Système de commande selon l'une des revendications 1 à 6, **caractérisé en ce que** le module d'analyse de forme d'onde (16) comporte une unité de comptage (20) servant à compter des maxima de pression ($P_1$, ...) à l'intérieur d'une onde de pression intracrânienne (22).

8. Système de commande selon l'une des revendications 1 à 7, **caractérisé en ce que** le moyen d'évaluation (12) est conçu comme un calculateur numérique binaire de telle sorte que des valeurs limites de pression et/ou de temps utilisées dans l'analyse se présentent sous la forme de puissances de deux de valeurs de référence limites respectives.

9. Système de commande selon l'une des revendications 1 à 8, **caractérisé par** un capteur de position (28) servant à détecter la position du patient qui communique avec le module de génération de signal de commande (21) par une liaison de signal.

10. Système de drainage (7), comprenant

    - un système de commande de drainage (9) selon l'une des revendications 1 à 9,
    - un moyen de commande de drainage (10) communiquant avec le module de génération de signal de commande (12) par une liaison de signal,
    - un moyen de drainage (8) destiné à être utilisé dans le conduit de drainage (3) pour guider le liquide hors de la boîte crânienne (2) du patient,
    - le dispositif de drainage (8) communiquant avec le moyen de commande de drainage (10) par une liaison de signal.

11. Système de drainage selon la revendication 10, **caractérisé par** un capteur d'écoulement servant à détecter un écoulement de fluide à travers le moyen de drainage (8).

12. Système de drainage selon la revendication 10 ou 11, **caractérisé en ce que** le capteur de pression (11) communique avec le moyen de drainage (8) et le moyen de commande de drainage (10) par une liaison de signal.

13. Système de drainage selon l'une des revendications 10 à 12, **caractérisé en ce que** le moyen de drainage (8) se présente sous la forme d'une soupape.

14. Système de drainage selon l'une des revendications 10 à 13, **caractérisé en ce que** le moyen de drainage (8) se présente sous la forme d'une pompe.

4

7

8

3

5

2

11

1

6

Fig. 1

Fig. 2

Fig. 3

Fig. 4

**EP 2 700 354 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2221083 A
- US 6248080 B1
- EP 0982048 A1
- DE 19643782 C1
- US 20070276264 A1